(19)

Europäisches
Patentamt
European
Patent Office
Office européen
des brevets

(11) **EP 2 439 185 A1**

(12) **EUROPEAN PATENT APPLICATION**

(43) Date of publication:
**11.04.2012 Bulletin 2012/15**

(21) Application number: **10173561.1**

(22) Date of filing: **20.08.2010**

(51) Int Cl.:
*C07C 1/04* (2006.01)          *B01J 23/889* (2006.01)
*B01J 29/076* (2006.01)      *B01J 29/26* (2006.01)
*B01J 37/03* (2006.01)        *C07C 11/02* (2006.01)
*B01J 37/02* (2006.01)

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Shell Internationale Research
Maatschappij B.V.
2596 HR The Hague (NL)**

(72) Inventor: **Verhaak, Michael Johannes Franciscus
Maria
1031 HW Amsterdam (NL)**

(74) Representative: **Matthezing, Robert Maarten et al
Shell International B.V.
Intellectual Property Services
P.O. Box 384
NL-2501 CJ Den Haag (NL)**

(54) **Process for preparing olefins from synthesis gas using a cobalt and manganese containing catalyst**

(57)    The invention relates to a process for preparing olefins from synthesis gas, wherein the synthesis gas is contacted with a catalyst which contains cobalt, manganese and a third element selected from the group consisting of alkali metals and earth alkaline metals. Further, the invention relates to a process for preparing such catalyst, and to the catalyst so obtained.

EP 2 439 185 A1

**Description**

The present invention relates to a process for preparing olefins from synthesis gas (syngas) using a catalyst which contains cobalt and manganese.

Syngas is a gas mixture which comprises hydrogen ($H_2$) and carbon monoxide (CO). Syngas may be converted into hydrocarbons by a catalytic process which is usually referred to as the Fischer-Tropsch (FT) process. Catalysts which are commonly used in the FT process, contain iron (Fe), cobalt (Co), nickel (Ni) and/or ruthenium (Ru) as the catalytically active metal. This is for example described by Van der Laan et al. in Catal. Rev.-Sci. Eng., 41, 1999, p. 255 ff.

There is a special interest for the selective and direct production of ethylene, propylene and butylene ($C_2$-$C_4$ olefins) from syngas, which are important chemical hydrocarbon feedstocks. Cobalt-manganese (Co-Mn) catalysts have been shown to have a relatively high selectivity to these olefins. Mirzaei et al. disclose in Applied Catalysis A: General, 306, 2006, p. 98 ff., the preparation of precipitated cobalt manganese catalyst and the use thereof in producing lower olefins from syngas.

An object of the present invention is to find a cobalt and manganese containing catalyst which has good selectivity and/or activity in the preparation of olefins from syngas.

Surprisingly it was found that said object is achieved by adding a third element to the cobalt and manganese containing catalyst, namely an element selected from the group consisting of alkali metals and earth alkaline metals.

Accordingly, the present invention relates to a process for preparing olefins from synthesis gas, wherein the synthesis gas is contacted with a catalyst which contains cobalt, manganese and a third element selected from the group consisting of alkali metals and earth alkaline metals.

Preferably, the olefins to be produced in the present process are light olefins, suitably unbranched $C_2$-$C_6$ alkenes, more preferably unbranched $C_2$-$C_4$ alkenes, and most preferably ethylene and propylene.

The catalyst to be used in the present process contains cobalt and manganese. In addition, it contains a third element, namely an element selected from the group consisting of alkali metals and earth alkaline metals. Examples of suitable earth alkaline metals are calcium (Ca) and magnesium (Mg). Mg is the preferred earth alkaline metal.

The atomic ratio of manganese to cobalt in the catalyst to be used in the present process may vary within wide limits. It may be of from 20:1 to 1:20, preferably 10:1 to 1:10, more preferably 10:1 to 1:2, most preferably 8:1 to 1:1.

The amount of the third element in the catalyst to be used in the present process may also vary within wide limits. It may be of from 0.01 to 5 wt.%, preferably 0.05 to 2.5 wt.%, more preferably 0.1 to 2 wt.%, even more preferably 0.2 to 1 wt.%, most preferably 0.3 to 0.8 wt.% based on total amount of the catalyst including any support.

Preferably, the catalyst to be used in the present invention is a supported catalyst. Examples of suitable supports for the present catalyst are titania ($TiO_2$), silica ($SiO_2$), alumina ($Al_2O_3$) and zeolite. A suitable example of such zeolite support is H-mordenite.

The catalyst to be used in the present invention may be prepared in a variety of ways. A supported catalyst may be prepared by means of a process comprising co-precipitation of a manganese salt and a cobalt salt on a support, followed by impregnation with a salt of the third element.

Accordingly, the present invention also relates to a process for preparing a supported catalyst, suitably for use in preparing olefins from synthesis gas, wherein the catalyst contains cobalt, manganese and a third element selected from the group consisting of alkali metals and earth alkaline metals, preferably magnesium, and is prepared by co-precipitation of a manganese salt and a cobalt salt on a support, followed by impregnation with a salt of the third element.

Preferably, said process for preparing a supported catalyst comprises combining the support with an aqueous solution containing the manganese salt and the cobalt salt, or with an aqueous solution containing the manganese salt and another aqueous solution containing the cobalt salt, resulting in a dispersion, and contacting the dispersion with a basic solution to effect the co-precipitation, and contacting the solid (precipitate) from the dispersion with an impregnating solution containing the salt of the third element.

Said manganese salt may be manganese nitrate. Said cobalt salt may be cobalt nitrate. Preferably, said solution or solutions containing the manganese salt and the cobalt salt is or are heated before combining with the support, for example at 50 to 90 °C, preferably 60 to 80 °C. The basic solution to be contacted with the dispersion may be a sodium carbonate solution. Preferably, said basic solution is heated before contacting with the dispersion, for example at 50 to 90 °C, preferably 60 to 80 °C. The amount of base in said solution should be sufficient to allow for co-precipitation of the manganese and cobalt salts on the support. This may be achieved by adding such amount of base that a pH of 8 or greater, preferably a pH of 9 or greater and most preferably a pH of 9.5 or greater is achieved in the dispersion. The solid (precipitate) in the dispersion or slurry resulting from the addition of the basic solution can be removed therefrom by filtration. The separated solid may be washed with water in order to remove substantially all base from the basic solution. Possibly after drying the separated solid, for example at a temperature in the range of from 80 to 120 °C, it may further be calcined in the presence of air, at a temperature in the range of from 200 to 800 °C, preferably 300 to 700 °C, more preferably 400 to 600 °C, most preferably 450 to 550 °C.

The above-mentioned separated solid is then to be impregnated with the salt of the third element, for example by contacting with an impregnating solution containing the salt of the third element, preferably magnesium. Said salt of the third element is any soluble salt, such as a nitrate, sulfate, chloride, hydroxide or organic acid salt of said third element. Preferably, said salt of the third element is a nitrate, sulfate, chloride, hydroxide or organic acid salt of magnesium. The paste that results from contacting the above-mentioned separated solid with an impregnating solution containing the salt of the third element may be heated for example at 40 to 80 °C, preferably 50 to 70 °C. Possibly after drying the impregnated catalyst, for example at a temperature in the range of from 80 to 120 °C, it may further be calcined in the presence of air, at a temperature in the range of from 200 to 800 °C, preferably 300 to 700 °C, more preferably 400 to 600 °C, most preferably 450 to 550 °C.

The present invention also relates to a catalyst obtainable by the catalyst preparation process(es) as described above.

Further, the present invention relates to a catalyst which contains cobalt, manganese and a third element selected from the group consisting of alkali metals and earth alkaline metals, preferably magnesium. Other preferences and embodiments regarding the catalyst from the catalyst preparation process and from the process for preparing olefins from synthesis gas as described above, also apply to said catalyst as such.

Before use in the present process for preparing olefins from synthesis gas, the catalyst may be reduced. Preferably, such reduction is performed by subjecting the catalyst to a gas stream comprising hydrogen and/or carbon monoxide. In addition, said gas stream may comprise nitrogen. The catalyst may for example be reduced at a temperature within the range of from 200 to 700 °C, suitably 300 to 600 °C, and preferably 400 to 500 °C.

The catalyst may be provided to a syngas conversion reactor as a fixed bed, a fluidized bed, or a slurry.

The synthesis gas to be used in the present process for preparing olefins from synthesis gas can be produced by generally known processes, as described for example in Weissermel et al., Industrial Organic Chemistry, Wiley-VCH, Weinheim, 2003, p. 15-24. For example, syngas can be produced by reaction of coal or methane with steam or by reaction of methane with carbon dioxide. Syngas usually has a volume ratio of carbon monoxide to hydrogen of from 3:1 to 1:3. In the present process, preferably a syngas is used which has a volume ratio of carbon monoxide to hydrogen of from 1:0.5 to 1:2.5.

The reaction temperature may be in the range of from 100 to 700 °C, suitably 200 to 600 °C, and preferably 250 to 500 °C, and most preferably 275 to 375 °C. The pressure may be of from 1 to 60 bar, preferably of from 10 to 50 bar. The GHSV (gas hourly space velocity) is generally from 100 to 30,000 parts by volume of feed stream per part by volume of catalyst per hour, in l/(l*hr) or hr$^{-1}$.

The invention is further illustrated by the following Examples.

Examples

Catalyst preparation

A catalyst containing cobalt and manganese with H-mordenite as support, hereinafter referred to as base-case catalyst, was prepared by means of a co-precipitation procedure.

Manganese nitrate (Mn (NO$_3$)$_2$ . 6H$_2$O) and cobalt nitrate (Co(NO$_3$)$_2$ . 6H$_2$O) were dissolved in demineralized water. The amounts of cobalt nitrate and manganese nitrate were chosen such that the Mn/Co atomic ratio in the final catalyst was 6.

The resulting aqueous solution was heated to 70 °C. H-mordenite was then added to the aqueous solution while stirring to obtain a dispersion. The amount of H-mordenite was chosen such that the weight percentage of H-mordenite was 15 wt.%, on the basis of the total weight of cobalt, manganese and H-mordenite.

In a separate vessel, sodium carbonate was dissolved in demineralized water to obtain a 1.0 M (mole/l) solution which was also heated to 70 °C. Basic carbonate solution was then added to the dispersion while stirring to achieve a final pH of about 10.

The slurry obtained was left stirring for another 30 minutes and then filtered. The solid residue was washed with warm demineralized water until free of sodium ions, as determined by atomic adsorption analysis. The solids were then dried at 110 °C for 16 hours resulting in a catalyst herein referred to as Reference Catalyst A.

A promoted catalyst was then prepared from the above base-case catalyst, containing cobalt and manganese with H-mordenite as support, by means of an impregnation procedure.

Magnesium nitrate was dissolved in demineralized water. The amount of magnesium nitrate was chosen such that the weight percentage of magnesium was 0.5 wt.%, on the basis of the total weight of magnesium and base-case catalyst. The base-case catalyst was impregnated with the magnesium nitrate solution resulting in a thick paste. The paste was heated to 60 °C in an oven and regularly stirred to homogenize the paste and finally it was left to dry for 16 hours. The dried material was calcined at 500 °C in air (heating rate 2 °C/minute), resulting in a catalyst herein referred to as Catalyst B.

Use of catalysts in syngas conversion

The above Reference Catalyst A (unpromoted) and Catalyst B (promoted with 0.5 wt.% of Mg) were then evaluated in the conversion of syngas in a small-scale, continuous flow, fixed bed setup. Prior to exposure to syngas, these catalysts were reduced in a flow of 50 vol.% hydrogen in nitrogen for 5 hours at a pre-reducing temperature of 350 °C (Reference Catalyst A1 and Catalyst B1) or 450 °C (Reference Catalyst A2 and Catalyst B2).

The catalyst was then cooled down, in the presence of said reducing gas, to 300 °C. Then the gas stream was switched to a stream of a mixture of carbon monoxide and hydrogen diluted in nitrogen. The overall gas composition of the latter stream was 33:17:50 (in vol.%) for $CO:H_2:N_2$ ($H_2/CO$ volume ratio was 0.5). The system was pressurized to 15 bar gauge (barg). The pressure and temperature during the reaction were maintained at 15 barg and 300 °C, respectively. The gas hourly space velocity (GHSV) was 1,000 $hr^{-1}$.

The product gas stream was analyzed with a gas chromatograph (GC) equipped with a flame ionization detector (FID) and a thermal conductivity detector (TCD). Table 1 shows the CO conversion (indicated as "CO") and the selectivities to carbon dioxide (indicated as "$CO_2$") and to the various hydrocarbon products (indicated by the molecular formula of the hydrocarbon product in question) as obtained in syngas conversions using Reference Catalysts A1 and A2 and Catalysts B1 and B2, at about 70 hours after the introduction of the syngas stream.

CO conversion is herein defined as:

$$((\text{moles CO}_{in} - \text{moles CO}_{out})/\text{moles CO}_{in})*100\%$$

Selectivity to $CO_2$ is herein defined as:

$$(\text{moles CO}_2)/(\text{moles converted CO})*100\%$$

Selectivity to hydrocarbon product "x" is herein defined as:

$$((\text{weight product "x"})/\text{total weight of all hydrocarbon}$$
$$\text{products excluding carbon dioxide})*100\%$$

Table 1

| Catalyst | CO | $CO_2$ | $CH_4$ | $C_2H_4$ olefin | $C_2H_6$ | $C_3H_6$ olefin | $C_3H_8$ | $C_4H_8$ olefin | $C_4H_{10}$ | $C_{5+}$ | Total $C_2$-$C_4$ olefins |
|---|---|---|---|---|---|---|---|---|---|---|---|
| A1 | 49.1 | 47.8 | 13.7 | 13.5 | 2.4 | 23.5 | 0.8 | 13.8 | 0.5 | 31.8 | 50.8 |
| B1 | 57.9 | 40.3 | 14.6 | 12.8 | 3.0 | 23.9 | 0.8 | 14.3 | 0.9 | 29.7 | 51.0 |
| A2 | 48.6 | 46.9 | 13.4 | 14.1 | 2.5 | 25.9 | 0.9 | 15.3 | 0.6 | 27.3 | 55.3 |
| B2 | 54.9 | 46.9 | 14.3 | 13.2 | 3.2 | 26.0 | 1.3 | 14.9 | 1.2 | 25.9 | 54.1 |

Upon comparing the results from Table 1 between the A1 and B1 catalysts (pre-reduction temperature of 350 °C) and between the A2 and B2 catalysts (pre-reduction temperature of 450 °C), respectively, it appears that the CO conversion increases significantly because of the presence of magnesium in the B catalysts.

Further, upon comparing the results from Table 1 between the A1 and B2 catalysts, it appears that both the CO conversion and the total selectivity to $C_2$-$C_4$ olefins, and in particular the selectivity to propylene ($C_3H_6$), increase significantly because of the presence of magnesium in the B2 catalyst in combination with the higher pre-reduction temperature for the B2 catalyst.

**Claims**

1. Process for preparing olefins from synthesis gas, wherein the synthesis gas is contacted with a catalyst which contains cobalt, manganese and a third element selected from the group consisting of alkali metals and earth alkaline metals.

2. Process according to claim 1, wherein the catalyst contains cobalt, manganese and magnesium.

3. Process according to claim 1 or 2, wherein the catalyst is reduced at a temperature within the range of from 400 to 500 °C.

4. Process for preparing a supported catalyst, wherein the catalyst contains cobalt, manganese and a third element selected from the group consisting of alkali metals and earth alkaline metals, and is prepared by co-precipitation of a manganese salt and a cobalt salt on a support, followed by impregnation with a salt of the third element.

5. Process according to claim 4, wherein the catalyst contains cobalt, manganese and magnesium.

6. Catalyst obtainable by the process of claim 4 or 5.

7. Catalyst which contains cobalt, manganese and a third element selected from the group consisting of alkali metals and earth alkaline metals.

8. Catalyst according to claim 7, which contains cobalt, manganese and magnesium.

Europäisches
Patentamt

European
Patent Office

Office européen
des brevets

# EUROPEAN SEARCH REPORT

Application Number

EP 10 17 3561

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | COLLEY S., BETTS M., COPPERTHWAITE R., HUTCHINGS G., COVILLE N.: "Carbon monoxide hydrogenation using cobalt-manganese oxide catalysts : The influence of potassium as a promoter" JOURNAL OF CATALYSIS, vol. 134, 1 January 1992 (1992-01-01), pages 186-203, XP002602731 * abstract * * page 187, column 1 - page 188, column 1 * * page 189, column 1 - page 191, column 1; figure 1; table 1 * | 1,3,7 | INV. C07C1/04 B01J23/889 B01J29/076 B01J29/26 B01J37/03 C07C11/02 B01J37/02 |
| X<br>Y | US 4 778 826 A (JEZL JAMES L [US] ET AL) 18 October 1988 (1988-10-18) * column 19, line 66 - column 21 * | 1-3,7,8<br>4-6 | |
| X | US 5 502 019 A (AUGUSTINE ROBERT L [US] ET AL) 26 March 1996 (1996-03-26) * column 2, line 18 - line 27 * | 7,8 | |
| X | US 5 371 306 A (WOO SEONG-IHL [KR] ET AL) 6 December 1994 (1994-12-06) * example 7; table 5 * | 7,8 | TECHNICAL FIELDS SEARCHED (IPC)<br><br>C07C<br>B01J |
| X | WO 2009/036877 A2 (BAYER MATERIALSCIENCE AG [DE]; MEYER HELMUT [DE]; HOCKE HEIKO [DE]; WE) 26 March 2009 (2009-03-26) * example 1 * | 7,8 | |
| X | WO 2007/093337 A2 (BAYER MATERIALSCIENCE AG [DE]; BUCHHOLZ SIGURD [DE]; MICHELE VOLKER [D) 23 August 2007 (2007-08-23) * example 1 * | 7,8 | |

-/--

The present search report has been drawn up for all claims

| Place of search | Date of completion of the search | Examiner |
|---|---|---|
| The Hague | 1 October 2010 | Patteux, Claudine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 10 17 3561

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 452 231 A1 (DIA NITRIX CO LTD [JP]) 1 September 2004 (2004-09-01) * example 7 * | 7 | |
| X | DENT A L ET AL: "Cobalt-Based Catalysts for the Production of C2-C4 Hydrocarbons from Syn-Gas" ADVANCES IN CHEMISTRY SERIES, AMERICAN CHEMICAL SOCIETY, WASHINGTON, DC, US, vol. 178, 1 June 1979 (1979-06-01), pages 47-63, XP008127550 ISSN: 0065-2393 * page 48 * * page 50 - page 51; table 1 * * figures 3b, 3d * | 1,3,7 | |
| Y | WO 03/043734 A1 (SASOL TECH PTY LTD [ZA]; VISAGIE JACOBUS LUCAS [ZA]; VAN ZYL ANDRE JOH) 30 May 2003 (2003-05-30) * page 4, line 1 - line 25 * | 4-6 | |
| Y | WO 2009/094935 A1 (ACCELERGY SHANGHAI R & D CT CO [CN]; BP INTERNAT LTD [GB]; BIAN GUOZHU) 6 August 2009 (2009-08-06) * figure 1 * | 4-6 | TECHNICAL FIELDS SEARCHED (IPC) |

The present search report has been drawn up for all claims

| Place of search | Date of completion of the search | Examiner |
|---|---|---|
| The Hague | 1 October 2010 | Patteux, Claudine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 17 3561

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2010

| Patent document cited in search report | | Publication date | Patent family member(s) | | Publication date |
|---|---|---|---|---|---|
| US 4778826 | A | 18-10-1988 | NONE | | |
| US 5502019 | A | 26-03-1996 | NONE | | |
| US 5371306 | A | 06-12-1994 | NONE | | |
| WO 2009036877 | A2 | 26-03-2009 | CN 101801394 A | | 11-08-2010 |
| | | | DE 102007044031 A1 | | 19-03-2009 |
| | | | EP 2190443 A2 | | 02-06-2010 |
| | | | KR 20100058544 A | | 03-06-2010 |
| | | | US 2009124705 A1 | | 14-05-2009 |
| WO 2007093337 | A2 | 23-08-2007 | CN 101384358 A | | 11-03-2009 |
| | | | DE 102006007147 A1 | | 23-08-2007 |
| | | | EP 1986775 A2 | | 05-11-2008 |
| | | | JP 2009526726 T | | 23-07-2009 |
| | | | KR 20080094690 A | | 23-10-2008 |
| | | | US 2008003169 A1 | | 03-01-2008 |
| EP 1452231 | A1 | 01-09-2004 | CN 1568223 A | | 19-01-2005 |
| | | | WO 03033139 A1 | | 24-04-2003 |
| | | | JP 4030740 B2 | | 09-01-2008 |
| | | | JP 2003117397 A | | 22-04-2003 |
| | | | RO 122022 B1 | | 28-11-2008 |
| | | | US 2004248734 A1 | | 09-12-2004 |
| WO 03043734 | A1 | 30-05-2003 | AU 2002347460 A1 | | 10-06-2003 |
| | | | CN 1589176 A | | 02-03-2005 |
| WO 2009094935 | A1 | 06-08-2009 | AU 2009208262 A1 | | 06-08-2009 |
| | | | CN 101559372 A | | 21-10-2009 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **Van der Laan et al.** *Catal. Rev.-Sci. Eng.,* 1999, vol. 41, 255 ff
- **Mirzaei et al.** *Applied Catalysis A: General,* 2006, vol. 306, 98 ff
- **Weissermel et al.** Industrial Organic Chemistry. Wiley-VCH, 2003, 15-24